(19) Europäisches Patentamt
European Patent Office
Office européen des brevets

(11) Publication number : **0 436 579 B1**

# (12) EUROPEAN PATENT SPECIFICATION

(45) Date of publication of patent specification : **04.08.93 Bulletin 93/31**

(51) Int. Cl.⁵ : **A61K 49/04,** A61K 49/00

(21) Application number : **89910435.0**

(22) Date of filing : **26.09.89**

(86) International application number : **PCT/EP89/01123**

(87) International publication number : **WO 90/03804 19.04.90 Gazette 90/09**

## (54) CHELATE COMPOSITIONS.

(30) Priority : **27.09.88 US 249745**
**27.09.88 US 249746**
**23.02.89 US 314729**
**28.02.89 US 317222**
**12.07.89 US 378776**
**31.07.89 US 386807**

(43) Date of publication of application : **17.07.91 Bulletin 91/29**

(45) Publication of the grant of the patent : **04.08.93 Bulletin 93/31**

(84) Designated Contracting States : **AT BE CH DE FR GB IT LI LU NL SE**

(56) References cited :
**EP-A- 263 059**
**WO-A-86/02352**

(56) References cited :
**WO-A-89/00052**
**DE-A- 3 401 052**
**DE-A- 3 633 243**
**US-A- 4 478 816**

(73) Proprietor : **NYCOMED SALUTAR, INC.**
**428 Oakmead Parkway**
**Sunnyvale, California 94086 (US)**

(72) Inventor : **CACHERIS, William, P.**
**1524 Dory Lane**
**Texas 75061 (US)**
Inventor : **QUAY, Steven, C.**
**4 Knoll Vista**
**Atherton, CA 94027 (US)**

(74) Representative : **Cockbain, Julian, Dr.**
**Frank B. Dehn & Co. Imperial House 15-19, Kingsway**
**London WC2B 6UZ (GB)**

**EP 0 436 579 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to metal chelate compositions, in particular contrast agent compositions containing chelates of diethylenetriaminepentaacetic acid bisamides (DTPA-bisamides) with calcium and with paramagnetic metal ions, lanthanide ions or other heavy metal ions.

In diagnostic imaging, contrast agents are frequently used to enhance the image contrast, for example between different organs or between healthy and unhealthy tissue within the same organ. The nature and manner of operation of the contrast agents depend upon the nature of the imaging technique and the organ or tissue which is to be imaged.

Thus in magnetic resonance imaging (MRI), image contrast may be enhanced by introducing into the body zone being imaged a contrast agent which affects the nuclear spin reequilibration characteristics of the nuclei (generally water protons in body tissues or fluids) which are responsible for the magnetic resonance (MR) signals from which the MR images are generated.

In 1978 Lauterbur (see Lauterbur et al., pages 752-759 in "Electrons to Tissues - Frontiers of Biological Energetics", Vol. 1, edited by Dutton et al., Academic Press, NY, 1978) proposed the use of paramagnetic metal ions, such as Mn(II), as MRI contrast agents. More recently Schering AG (in EP-A-71564 and US-A-4647447) proposed the use of the dimeglumine salt of the gadolinium (III) chelate of diethylenetriaminepentaacetic acid (GdDTPA) as a MRI contrast agent. However, even though the stability contrast of the GdDTPA chelate is very high, this compound, following iv administration, still releases highly toxic gadoliniUm and thus its dosage limit and range of imaging applications is limited by toxicity factors.

Various DTPA derivatives have been suggested in the literature as the chelating agents for other paramagnetic metal chelate MRI contrast agents and several of these have been found to have lower toxicities than the DTPA chelates. Thus for example GdDTPA-bisamides, and in particular GdDTPA-bisalkylamides, described by Salutar Inc in US-A-4687659 and Gd DTPA-bis(hydroxylated-alkylamides), such as those described by Dean et al. in US-A-4826673 and by Schering AG in EP-A-130934, have been found to be substantially less toxic than GdDTPA-dimeglUmine. Such chelates may thus be used in substantially higher quantities than GdDTPA and so are useful in a wider range of imaging applications. However from even such chelate compounds as these there will be some in vivo release of the paramagnetic metal and there is thus a continuing need for the development of MRI contrast media of lower toxicity.

Metal chelates have also been suggested as contrast agents for X-ray imaging where contrast is achieved by altering the attenuation of X-radiation passing through the body being imaged. The X-ray attenuating effect increases generally with atomic number and elements such as lanthanides and heavy metals having atomic numbers above 50 can provide the contrast required for clear definition of body organs, body cavities, the vascular system etc. Consequently, although iodinated organic compounds are now widely used as X-ray contrast agents, lanthanide and other heavy metal compounds have attractive potential as X-ray contrast agents (see for example US-A-4310507).

These metals include Ba, Bi, Cs and lanthanides such as Ce, Dy, Lu, Yb and Gd for example. However their toxicity limits the usefulness of these metals, particularly for oral or parenteral administration. They could not hitherto be used safely at the dosages or concentrations required for effective contrast enhancement. Moreover previously known lanthanide and heavy metal chelates, while showing stability in vitro show substantial and unacceptable toxicity after administration.

The conventional iodinated X-ray contrast agents however do have some limitations, particularly the significant decrease in X-ray attenuation with the high keV X-ray emissions required for computer tomography (CT) scanning. Moreover commercial iodinated compound formulations generally have high viscosities and are not too easily administered. Moreover, while the toxicity levels of the iodinated X-ray contrast agents are generally acceptable such compounds can give rise to physiochemitoxic dose-related reactions and to dose-unrelated idiosyncratic reactions (see for example Brasch, "contrast media in paediatric radiology", Workshop, Berlin, August 22-24, 1985, NY, Karger (1987)).

Seltzer et al. have reported in Invest. Radiol. 14:400 (1979) that elements with atomic numbers between 58 and 68 attenuate a 120 kVp X-ray beam more than elements with higher or lower atomic numbers and thus compounds of such elements have potential as X-ray contrast agents. The use of lanthanide series oxides, e.g. cerium, gadolinium and dysprosium oxides, as contrast agents in CT scanning of the liver using a 120 kVp polyenergetic X-ray beam was reported by Havron et al. in J. Comput. Assist. Tomogr. 4:642-648 (1981). Similar experiments were reported by Seltzer et al. in J. Comput. Assist. Tomogr. 5:370-374 (1981).

US-A-4478816 describes methods of digital radiography using as contrast agents rare earth chelates of diethylenetriaminepentaacetic acid (DTPA), ethylenediamine-N,N'-bis(2-hydroxyphenylacetic acid) (EHPG) and N,N'-bis(2-hydroxybenzyl)ethylenediamine-N,N'-diacetic acid (HBED). Lutetium and yttrium chelates are said to be particularly useful in vascular diagnostic studies by digital fluoroscopy because of their K-edge mass

attenuation coefficient discontinuities at a high energy level near 60 keV. US-A-4647447 supra also describes metal chelates as X-ray contrast agents and US-A-4176173 describes tantalum and hafnium chelates which can be used as contrast agents with high energy X-ray sources, particularly in imaging studies of the intestines.

The X-ray applications at high energy levels such as CT scanning would benefit from better contrast agents. The greatest relative ratio of contrast (contrast iodine/soft tissue) will be attained when the energy of the X-ray beams straddles the large mass attenuation coefficient discontinuity at the K-edge. However, for all iodinated contrast agents, consideration of excessive exposure to the patient and insufficient X-ray tube output intensity at low energy levels (near 33 kVp) negate utilization of the K-edge subtraction approach. Unfortunately, iodine compounds do not provide the necessary attenuation for high kVp beams, and do not provide the level of image improvement needed.

A safe and effective form for administering lanthanides and heavy metals for X-ray contrast imaging is thus a very important need in this field.

Thus although the use of paramagnetic metal chelates and chelates of lanthanides and other heavy metals as contrast agents in diagnostic imaging, especially X-ray imaging, is known and widely described, the major X-ray contrast agents continue to be the iodinated compounds not least because of the toxicity problems associated with the metals.

A summary of the pharmacology and toxicology of the lanthanides is provided for example by Haley in J. Pharm. Sci. 54:663-670 (1965).

While the toxicity of the metal species is generally significantly reduced by formulation as a chelate compound and while many chelates have improved solubilities relative to the free metal oxides, as mentioned above the problem of metal toxicity remains even with high stability constant chelates since undesirable levels of the toxic metal ions are released from the chelate complex into the body after administration. The concentrations at which metal chelates may be administered as contrast agents, and hence the utility of such chelates as contrast agents are thus limited by toxicity constraints. Indeed for X-ray imaging the concentration of chelate considered safe for general use with human subjects may be insufficient for effective X-ray attenuation and for MR imaging the usefulness of even high stability constant chelates such as GdDTPA could be limited since the dosage required for imaging certain organs may possibly be too toxic for safe and effective use.

It has therefore been an objective within the field of diagnostic imaging, and especially X-ray and MR imaging, to develop metal chelate containing contrast media of reduced toxicity.

One approach, as mentioned above, has been to investigate different chelating agents and in the MRI field for example there are numerous publications proposing the use of many different complexing agents for chelating the paramagnetic metal ions. Examples of such pulications include WO89/00557 (Nycomed AS), EP-A-292689 (Squibb), EP-A-232751 (Squibb), EP-A-230893 (Bracco), EP-A-255471 (Schering), EP-A-277088 (Schering), EP-A-287465 (Guerbet), and the documents cited therein.

An alternative approach has been to seek to enhance the bioacceptability of the contrast media by formulation of the contrast agent with other materials.

Thus in EP-A-270483, in which Schering acknowledge the problem of in vivo gadoliniUm release from GdDTPA, Schering have disclosed that they have found retention in the body of the heavy metal (i.e. gadolinium) from heavy metal complexes is reduced by formulating the heavy metal complex together with one or more weaker metal complexes and/or one or more free complexing agents. Schering have stated in this publication that the addition of free complexing agents or weak complexes has an extraordinarily strong effect with reference to the stability of the bonding of the heavy metal and thus its detoxification and elimination but this statement is qualified by a reference to the effect that a higher toxicity may need to be accepted as the price for this advantage.

In general the inclusion of excess chelating agents in metal chelate containing contrast media is known from the literature.

The present invention is based on our surprising discovery that for metal chelate based contrast media, by using a DTPA-bisamide as the chelating entity and by including within the contrast media a calcium DTPA-bisamide chelate an unpredictably large reduction in the acute toxicity of the contrast medium can be obtained thus enabling contrast media, e.g. MRI, X-ray scintigraphic and ultrasound contrast media, of significantly reduced toxicity to be produced and thereby extending the potential field of use of metal chelates as contrast media, especially in MRI and X-ray imaging.

Viewed from one aspect therefore the present invention provides a diagnostic imaging contrast medium comprising a chelate of a 6-carboxymethyl-3,9-bis(carbamoylmethyl)-3,6,9-triazaundecanedioic acid (hereinafter a DTPA-bisamide) with a cation (hereinafter the imaging cation) selected from paramagnetic metal ions, lanthanide ions and heavy metal ions, and a toxicity reducing amount of a calcium chelate of a DTPA-bisamide, said acids not carrying any alkoxyalkyl substituents.

Preferably, the chelates in the contrast media of the invention are chelates with compounds of formula I

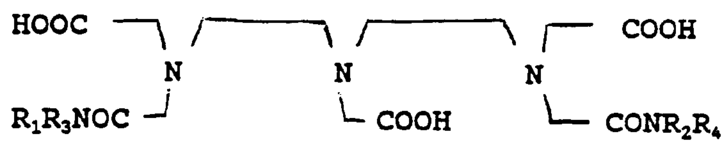

(I)

wherein $R_1$ to $R_4$ are each, independently, hydrogen, lower (e.g. $C_{1-6}$) alkyl, hydroxy lower alkyl, or polyhydroxy $C_{1-18}$ alkyl.

In the definition of substituents $R_1$ to $R_4$ above, alkyl is deemed to include straight and branched as well as unsaturated and, preferably, saturated groups.

Preferred DTPA-bisamides of formula I include those of formula Ia

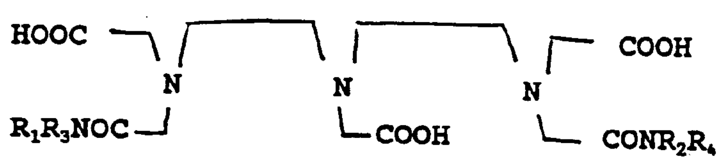

(Ia)

wherein $R_3$ and $R_4$ are hydrogen and $R_1$ and $R_2$ are as defined above, especially hydrogen or lower alkyl (particularly methyl), as well as those of formula Ib

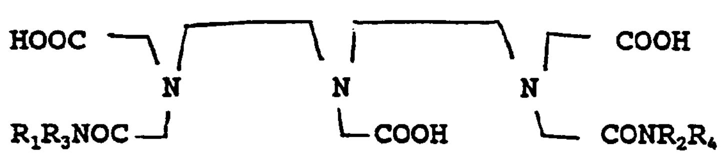

(Ib)

wherein $R_1$ and $R_2$ are independently $C_{1-6}$ mono- or polyhydroxyalkyl and $R_3$ and $R_4$ are independently hydrogen or $C_{1-6}$, alkyl, especially methyl. Particularly preferred DTPA-bisamides of formula I include those of formula Ic

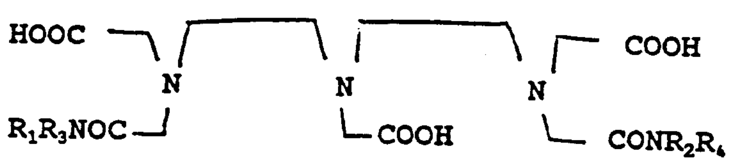

(Ic)

wherein $R_1$ and $R_2$ both represent hydrogen or dihydroxypropyl and $R_3$ and $R_4$ both represent hydrogen or methyl. Especially preferred DTPA-bisamides of formula I include
6-carboxymethyl-3,9-bis(methylcarbamoylmethyl)-3,6,9-triazaundecanedioic acid (DTPA-BMA);
6-carboxymethyl-3,9-bis(N-methyl-N-2,3-dihydroxypropylcarbamoylmethyl)-3,6,9-triazaundecanedioic acid (DTPA-BMPA); and
6-carboxymethyl-3,9-bis(2,3-dihydroxypropyl-carbamoylmethyl)-3,6,9-triazaundecanedioic acid (DTPA-APD).

Preferably the calcium chelate and the paramagnetic/lanthanide/heavy metal chelate are both chelates of DTPA-bisamides of formula Ia or are both chelates of DTPA-bisamides of formula Ib or are both chelates of DTPA-bisamides of formula Ic. Especially preferably the calcium and paramagnetic/lanthanide/heavy metal chelates are both chelates of the same DTPA-bisamide.

The contrast media of the invention may be used in various different types of diagnostic imaging, for example MR, X-ray, ultrasound and scintigraphic imaging, but are especially suited for use as MRI or X-ray contrast media. In this regard it will be realised that the choice of the imaging cation will of course depend upon the nature of the imaging technique in which the contrast medium is intended to be used.

Thus for MRI contrast media the imaging cation will be a paramagnetic metal ion, preferably a non-radioactive species, and conveniently the metal will be a transition metal or a lanthanide, preferably having an atomic number of 21-29, 42, 44 or 57-71. Metal chelates in which the metal species is Eu, Gd, Dy, Ho, Cr or Fe are especially preferred and $Gd^{3+}$ and $Dy^{3+}$ are particularly preferred.

In the case of X-ray and ultrasound contrast media, the imaging cation is preferably a cation of a lanthanide or heavy metal species, e.g. a non-radioactive metal, with an atomic number greater than 37, preferably 50 or more, e.g. Ce, Dy, Er, Eu, Au, Ho, La, Lu, Hg, Nd, Pr, Pm, Sm, Tb, Th and Yb. Preferred metals include La, Yb, Dy and Gd, and $Dy^{3+}$ and $Gd^{3+}$ are especially preferred.

For scintigraphic contrast media, the imaging cation must of course be radioactive and any conventional radioactive metal isotope complexable by a DTPA-bisamide may be used.

It is especially preferred that the chelate complexes, particularly those of the imaging cations, be neutral, that is the positive charge on the cation should be matched by an equal negative charge on the chelating moiety. Thus in the case of chelates of DTPA-bisamides of formula I, the imaging cation is preferably an $M^{3+}$ cation, as is the case for example with gadolinium (III) or dysprosium (III).

Without being limited to a particular theory of interaction which might underlay the surprising effectiveness of the contrast media of the invention, the principle toxicity of the metal chelate contrast agents is believed to derive from the displacement of the imaging cation from the chelate by endogeneous metal ions, especially Zn(II), normally present in the body. It is believed that when contrast media according to the invention are administered the Zn(II) available for displacement preferentially displaces calcium from the calcium DTPA-bisamide chelates, liberating non-toxic calcium into the blood and leaving the imaging cation secure in a stable chelate. The calcium DTPA-bisamide chelates have been found to be uniquely suitable for improving toxicity, apparently because they act in a unique manner in this interaction. Administration of calcium chelates of chelating agents other than DTPA-bisamides (e.g. CaDTPA) and administration of calcium DTPA-bisamide chelates with chelates of imaging cations with chelating agents other than DTPA-bisamides (e.g. Gd DTPA) has not been found to produce the surprising improvements in toxicity levels achieved with compositions according to the invention.

With the reduced toxicity provided by the contrast media of the invention, image contrast can be increased with greater safety and higher dosages of the contrast agent, i.e. of the imaging cation, can safely be administered, providing the ability to enhance image contrast in a wider range of imaging applications, e.g. for a,wider range of organs.

As mentioned above, it is thought that the toxicity reduction is linked to Zn(II) displacement of imaging cations and thus the imaging cations are conveniently cations, e.g. lanthanide or heavy metal cations, displaceable from a DTPA-bisamide chelate by Zn(II). Examples of metal ions falling within this definition include the ions of Ce, Dy, Er, Eu, Gd, Au, Ho, La, Lu, Hg, Nd, Pr, Pm, Sm, Tb, Th and Yb.

DTPA-bisamide chelating agents, such as those of formula I above, are either known from the literature or may be prepared in manners analogous to those described in the literature. Thus for example DTPA-bisalkylamides and methods for their preparation are disclosed in US-A-4687659 and DTPA-bis(hydroxyalkylamides) and methods for their preparation are disclosed in US-A-4826673 and EP-A-130934.

The metal chelates of the DTPA-bisamides, i.e. the chelates with the imaging cation and with calcium, can be prepared in a conventional manner, e.g. by mixing in aqueous solution stoichiometric quantities of a DTPA-bisamide and a compound of the metal which dissolves to liberate the metal species to be chelated, e.g. a metal oxide or hydroxide (such as calcium hydroxide or gadolinium oxide) or a soluble salt of calcium or of the imaging cation.

The calcium chelate in aqueous solution is preferably a fully neutralized salt. The counterion for the calcium chelate complex and indeed any necessary counterion for the imaging cation chelate complex can be any pharmaceutically acceptable non-toxic ion. Suitable counterions include monovalent cations such as ions of lithium, potassium and sodium and divalent cations such as calcium and magnesium can also be used. Suitable cations of organic bases include, for example, ethanolamine, diethanolamine, morpholine, glucamine, N,N-dimethylglucamine, and N-methylglucamine. Ions of amino acids such as lysine, arginine and ornithine and other basic naturally occuring acids may also be considered.

The contrast medium may be prepared by reacting a DTPA-bisamide with soluble calcium and paramagnetic metal, lanthanide or other heavy metal compounds in an aqueous medium and such a process forms a further aspect of the invention: preferably however the metal chelates will be prepared separately and then admixed in the desired ratio.

The contrast medium of the invention should contain a sufficient amount of the calcium DTPA-bisamide chelate to reduce the toxicity of the composition relative to that of a composition from which the calcium chelate is omitted but which is otherwise identical; however, any amount of the calcium chelate will generally provide some improvement. The amount of the calcium chelate present should however be below toxic limits. conveniently compositions have a molar ratio of calcium chelate to imaging ion chelate in the range from 1:200 to 1:5, preferably 1:200 to 1:10, especially 1:100 to 1:10, particularly 1:100 to 1:20, more particularly 1:30 to 1:15,

especially 1:20. The dosage amount of the calcium chelate can conveniently be at a physiologically tolerable level in the range of from 0.001 to 5 mmol/kg, preferably 0.001 to 1 mmol/kg, especially preferably from 0.004 to 0.08 mmol/kg of patient bodyweight.

As the calcium chelate may improve the biotolerability of the imaging ion chelate by hindering displacement of imaging ions from the chelate by zinc ions it may therefore be desirable to match the concentration or dosage of calcium chelate to the plasma zinc concentration of the subject to which or to whom the contrast medium is to be administered.

The concentration of the imaging ion chelate in the compositions of the invention may be at conventional levels or higher than conventional levels for the imaging technique. Generally the contrast media of the invention may contain from 0.001 to 5.0 moles per litre, preferably 0.1 to 2 moles/litre, especially 0.1 to 1.2 moles/litre and particularly 0.5 to 1.2 moles/litre of the imaging ion chelate. For MRI contrast media, paramagnetic metal chelate concentrations are conveniently 0.001 to 5, especially 0.1 to 1.2, mole/litre while for X-ray contrast media lanthanide or heavy metal chelate concentrations are conveniently 0.1 to 2, preferably 0.5 to 1.2, mole/litre.

The contrast media of the invention are administered to patients for imaging in amounts sufficient to yield the desired contrast with the particular imaging technique. Generally dosages of from 0.001 to 5.0 mmoles of imaging ion chelate per kilogram of patient bodyweight are effective to achieve adequate contrast enhancement. For most MRI applications preferred dosages of imaging ion chelate will be in the range from 0.02 to 1.2 mmoles/kg bodyweight while for X-ray applications dosages of from 0.5 to 1.5 mmoles/kg are generally effective to achieve X-ray attenuation. Preferred dosages for most X-ray applications are from 0.8 to 1.2 mmoles of the lanthanide or heavy metal chelate/kg bodyweight.

For general use in X-ray radiography with a lanthanide or heavy metal chelate, a dosage of at least 1 mmol/kg is required for optimum contrast. The ratio $LD_{50}$ to maximum effective dosage in rats considered to provide an adequate margin of safety is about 15. GdDTPA has an $LD_{50}$ of 5.5 mmol/kg. GdDTPA-bis (methylamide) (GdDTPA-BMA) has an $LD_{50}$ of 15 mmol/kg. The $LD_{50}$ of a mixture of CaNaDTPA-BMA and GdDTPA-BMA is 34.4 mmol/kg, well above the level required for effective X-ray image contrast.

The contrast media of the present invention may be formulated with conventional pharmaceutical or veterinary formulation aids, for example stabilizers antioxidants, osmolality adjusting agents, buffers and pH adjusting agents, and may be in a form suitable for parenteral or enteral administration, for example injection or infusion or administration directly into a body cavity having an external escape duct, for example the gastrointestinal tract, the bladder or the uterus. Thus the contrast media of the present invention may be in.conventional pharmaceutical administration forms such as tablets, capsules, powders, solutions, suspensions, dispersions, syrups and suppositories; however, solutions, suspensions and dispersions in physiologically acceptable carrier media, for example water for injections, will generally be preferred.

The contrast media according to the invention may therefore be formulated for administration using physiologically acceptable carriers or excipients in a manner fully within the skill of the art. For example, the chelate components, optionally with the addition of pharmaceutically acceptable excipients, may be suspended or dissolved in an aqueous medium, with the resulting solution or suspension then being sterilized. As mentioned above, suitable additives include, for example, physiologically biocompatible buffers (as for example, tromethamine hydrochloride), slight additions of other chelating agents (as for example, diethylenetriaminepentaacetic acid) or, optionally, calcium or sodium salts (for example, calcium chloride, calcium ascorbate, calcium gluconate or calcium lactate).

If the contrast media are to be formulated in suspension form, e.g. in water or physiological saline for oral administration, a small amount of soluble chelate salt may be mixed with one or more of the inactive ingredients traditionally present in oral solutions and/or surfactants and/or aromatics for flavouring.

For MRI and for X-ray imaging of some portions of the body the most preferred mode for administering metal chelates as contrast agents is parenteral, e.g. intravenous, administration. Parenterally administrable forms, e.g. intravenous solutions, should be sterile and free from physiologically unacceptable agents, and should have low osmolality to minimize irritation or other adverse effects upon administration and thus the contrast medium should preferably be isotonic or slightly hypertonic. Suitable vehicles include aqueous vehicles customarily.used for administering parenteral solutions such as Sodium chloride Injection, Ringer's Injection, Dextrose Injection, Dextrose and Sodium chloride Injection, dictated Ringer's Injection and other solutions such as are described in REMINGTON'S PHARMACEUTICAL SCIENCES, 15th ed., Easton: Mack Publishing co, pp 1405-1412 and 1461-1487 (1975) and THE NATIONAL FORMULARY XIV, 14th ed. Washington: American Pharmaceutical Association (1975). The solutions can contain preservatives, antimicrobial agents, buffers and antioxidants conventionally used in parenteral solutions, excipients and other additives which are compatible with the chelates and which will not interfere with the manufacture, storage or use of products.

The contrast media of the invention may also, of course, be in concentrated or dried form for dilution prior to administration.

Viewed from a yet further aspect the invention provides the use of a calcium DTPA-bisamide chelate for the preparation of a contrast medium according to the invention.

Viewed from a still further aspect the invention provides the use of a DTPA-bisamide for the preparation of a contrast medium according to the invention.

Viewed from another aspect the invention provides the use of a chelate of a DTPA-bisamide with a cation selected from paramagnetic metal cations, lanthanide cations and other heavy metal cations, for the preparation of a contrast medium according to the invention.

Viewed from a further aspect the invention provides a method of generating an image of a human or animal body, said method comprising administering to said body a contrast medium according to the invention and generating an image, e.g. an MR, X-ray, ultrasound, scintigraphic etc. image, of at least part of said body, e.g. after permitting sufficient time to elapse for the medium to distribute to the desired parts of said body.

Methods for applying MRI contrast media to improve MR images, MRI equipment and MRI operating procedures are described by Valk et al., BASIC PRINcIPLES OF NUCLEAR MAGNETIC RESONANCE IMAGING, New York: Elsevier, pp 109-114 (1985).

For X-ray imaging, the X-ray contrast media of the invention can be used in standard conventional X-ray procedures with conventional equipment in the same manner as other contrast media. For other imaging techniques, standard equipment and procedures may also be used.

With the contrast media of this invention, effective imaging contrast can be obtained with a wider variety of organs such as kidney, urethra, bladder, brain, spine, heart, liver, spleen, adrenal glands, ovaries and skeletal muscle.

The invention is further illustrated by the following examples and by the accompanying drawings in which Figures 1, 2 and 3 are plots of CT scans at 80 keV, 120 keV and 140 keV respectively of the Intensity/Metal centre vs. Concentration for iohexol, Gd DTPA-BMA and Dy DTPA-BMA.

Temperatures are given in degrees celsius and concentrations as weight percentages unless otherwise specified.

## EXAMPLE 1

### DTPA-bismethylamide Dihydrate

Methylamine (40% solution, 1.52 kg) in a 4 L beaker was cooled in an ice bath. Solid DTPA-bisanhydride (1.0 kg) was added over 30-60 minutes with vigorous stirring. The temperature was maintained below 35°C during the addition. Then the ice bath was removed, and the brown solution stirred at room temperature for two hours. The mixture was acidified to pH ca. 3 with concentrated Hcl, and the solution was filtered at 60-70°C to remove traces of insoluble material. To the hot solution was added 2.5 L ethanol and 2.5 L of isopropanol. After cooling to room temperature for 6-24 hours, a colourless mass of tan crystals was isolated by filtration. The filter cake was washed with ethanol and then redissolved in 3 L ethanol, 3 L isopropanol, and 3 L water. Active charcoal was added to the hot solution, followed by vacuum filtration. The solution was allowed to cool to room temperature. After 6-24 hours, brilliant white crystals were collected, washed with 2 L ethanol, and dried for 12-24 hours. After drying, 838 g (67% yield) of the title product were obtained, mp 110-113°C.

## EXAMPLE 2

### Gd DTPA-bismethylamide Trihydrate

DTPA-bismethylamide dihydrate (455.5 g, 1.0 mol), $Gd_2O_3$ (181.2 g, 0.50 mol) and water (750 ml) are combined and heated under reflux for 6-8 hours or until all solid dissolves. The mixture is then checked for the presence of free gadolinium with xylenol orange. If free gadolinium is present, another 5 g of amide is added, and the mixture heated under reflux for 2 hours. The xylenol orange test is repeated, and more amide added if necessary. Once the reaction mixture is clear with no free gadoliniUm present, it is cooled to room temperature, and the pH is checked. A small amount of 1 N NaOH can be added to adjust the pH of the mixture to 6.0-6.5. The solution is filtered, and an equal volume of acetone is added with stirring. After 4-6 hours, colorless crystals are collected, washed with 250 ml of acetone, and dried for 12-24 hours in vacuo (1 mmHg, 60-80°C).

A yield of 540 g (86%) of product Gd DTPA-BMA was obtained.

Replacing $Gd_2O_3$ in the above procedure by $Yb_2O_3$ or $La_2O_3$ the corresponding Yb or La chelates of DTPA-BMA are produced.

EXAMPLE 3

NaCa DTPA-bismethylamide

Repeating the procedure of Example 2, replacing the $Gd_2O_3$ oxide with a stoichiometric molar equivalent amount of caO yields the corresponding NaCa DTPA-BMA.

Naca DTPA-bisethylamide and Naca DTPA-bispropylamide are produced analogously using caO and DTPA-bisethylamide or DTPA-bispropylamide (see Examples 16 and 18).

EXAMPLE 4

Formulation of Gd DTPA-bismethylamide and NaCa DTPA-bismethylamide

Solid gadolinium DTPA-bismethylamide trihydrate (3.14 g, 5.0 mmol) was dissolved in ca. 6 mL of water with stirring. Sodium calcium DTPA-bismethylamide trihydrate (0.13 g, 0.25 mmol) was added, and the pH was 6.0. The addition of 10 microliters of 1 N NaOH (0.01 mmol, 0.2 mole%) increased the pH to 6.25. The colorless mixture was diluted to 10 mL and sterile filtered into a serum vial to yield a formulation that was 500 mM gadolinium DTPA-bismethylamide and 25 mM sodium calcium DTPA-bismethylamide.

Formulations containing Gd DTPA-bisethylamide and Naca DTPA-bisethylamide or Gd DTPA-bispropylamide and Naca DTPA-bispropylamide may be prepared analogously using the gadolinium and calcium chelates of Examples 17, 19 and 3. Compositions containing Naca DTPA-BMA or Naca DTPA-bisethylamide and the Yb or La chelates of DTPA-BMA or DTPA-bisethylamide may similarly be prepared using the chelates of Examples 2, 3 and 17.

EXAMPLE 5

DTPA-bis(2,3-dihydroxypropylamide) Dihydrate

A mixture of 54.8 g (600 mmol) of 3-amino-1,2-propanediol, 84 mL (600 mmol) triethylamine, and 200 mL dimethyl sulfoxide (DMSO) were combined with stirring at room temperature. DTPA anhydride (71.5 g, 200 mmol) was added in portions over 10 min. After stirring for 2 hrs at room temperature, the amber reaction mixture was concentrated under reduced pressure. The crude reaction product was adjusted to pH 3.5 with 6 N Hcl and then chromatographed on a column of AGI-X4 anion exchange resin (acetate form). The product was eluted with 1 N acetic acid to provide 103 g (89%) of DTPA-bis-(2,3-dihydroxypropylamide) dihydrate (DTPA-APD dihydrate) as an oil. Lyophilization provided a colorless solid which was determined to possess two waters of hydration.

EXAMPLE 6

Gd DTPA-APD

Gadolinium oxide (18.128 g, 50 mmol) and 54 g (94 mmol) DTPA-APD dihydrate were combined in 60 ml water and heated under reflux. Additional DTPA-APD dihydrate was added in small portions over 6 hours at reflux until a Xylenol orange test for excess gadoliniUm ion was negative. A total of 57.75 g (100 mmol) ligand was necessary. After cooling, the reaction mixture was diluted to 150 mL with water and filtered (0.2 micron membrane filter). This 667 mM Gd DTPA-APD solution was used for the formulations described in Examples 8 and 9.

EXAMPLE 7

NaCa DTPA-APD

DTPA-APD dihydrate (1.44 g, 2.5 mmol) and calcium hydroxide (186 mg, 2.5 mmol) were combined in 3 mL water and adjusted to pH 6.6 by the addition of ca. 1.5 mL 1 N NaOH solution. This was used directly for a formulation described in Example 9.

EXAMPLE 8

Preparation of 500 mM Gd DTPA-APD

A 67.5 mL (45 mmol) portion of the 667 mM Gd DTPA-APD stock solution described in Example 6 was adjusted to pH 6.0 with 1 N NaOH solution. After dilution to 90 mL, the resulting 500 mM Gd DTPA-APD solution was sterile-filtered into serum vials and autoclaved 30 minutes at 121°C.

EXAMPLE 9

Formulation of 500 mM Gd DTPA-APD and 25 mM Na Ca DTPA-APD Solution

A 75 mL (50 mmol) portion of the 667 mM Gd DTPA-APD stock solution described in Example 6 was combined with the Na Ca DTPA-APD preparation (2.5 mmol) of Example 7. The resulting solution was adjusted to pH 5.9 with 1N NaOH and diluted to 100 mL. The solution was sterile-filtered into serum vials and autoclaved 30 minutes at 121°C.

EXAMPLE 10

DTPA-bis(N-methyl-2,3-dihydroxypropylamide)

N-methylaminopropanediol (50.0 g, 139.9 mmol) was dissolved in DMSO (250 mL), and DTPA-bisanhydride was added under a nitrogen atmosphere. After stirring overnight (10 hours), the product was precipitated with a 1:1 mixture of ether and chloroform. The crystals were dissolved in water (150 mL) and precipitated once more with ethanol (1400 mL). After 1 hour, the product was separated from the solvent. Stirring with ethanol (600 mL) produced a white powder which was dried in vacuum. The yield of DTPA-bis-(N-methyl-2,3-dihydroxypropylamide) (DTPA-BMPA) was 45.0 g (56%). Mp. 75-80°C. FAB-MS: 568(M+1).

EXAMPLE 11

Gd DTPA-BMPA

DTPA-BMPA (39.0 g, 68.7 mmol) was dissolved in water (50 mL). The water was distilled off to remove ethanol; the oil was dissolved in water (250 mL); and $Gd_2O_3$ (11.2 g, 31.0 mmol) was added. The mixture was stirred for 16 hours at 100°C and filtered, and the solvent was removed. The product was dissolved in methanol (110 mL) and precipitated with acetone (250 mL). The product was dissolved in water and dried. This was repeated two times to remove traces of acetone. Yield 40.4 g (81%). Mp. 280°C. FAB-MS: 723(M+1).

| | | | |
|---|---|---|---|
| Anal. Calcd. for $C_{22}H_{38}GdN_5O_{12}$: | C, 35.60 | H, 5.31; | N, 9.70 |
| Found: | 36.12 | 5.25 | 10.39 |

EXAMPLE 12

NaCa DTPA-BMPA

DTPA-BMPA (5.0 g, 8.8 mmol) was dissolved in water (50 ml), and $Ca(OH)_2$ (0.65 g, 8.8 mmol) was added. The mixture was stirred at room temperature for about 1.5 hours. The solution was neutralized with 2 M NaOH and then filtered. The filtrate was evaporated to dryness, and the Naca DTPA-BMPA compound was isolated as a white powder. Yield 5.2 g (84%). Mp. 230-233°C. FAB-MS: 628(M+1)

| | | | |
|---|---|---|---|
| Anal. Calcd. for $C_{22}H_{38}CaN_5NaO_{12}$: | C, 42.10; | H, 6.10; | N,11.16 |
| Found: | 41.48 | 5.96 | 10.96 |

EXAMPLE 13

Gd DTPA-BMPA and NaCa DTPA-BMPA Solution

Gd DTPA-BMPA (3.61 g, 5 mmol) and Naca DTPA-BMPA (0.157 g, 0.25 mmol) were dissolved in water (7 mL). The pH was adjusted to between 5.5 and 6.5 and water (to 10 mL) was added. The solution was sterile filtered into a 10 mL vial. The solution contained 0.5 mmol of Gd per mL.

EXAMPLE 14

Dy DTPA-bismethylamide Trihydrate

DTPA-bismethylamide dihydrate (DTPA-BMA) (22.77 g, 50.0 mmol) prepared according to the procedure of Example 1, $Dy_2O_3$ (9.32 g, 25.0 mmol) and water (60 mL) are combined and heated under reflux for 6-8 hours or until all solids have dissolved. The mixture is tested for free dysprosium with xylenol orange. If free dysprosium is present, another 0.25 g of amide is added, and the mixture is heated under reflux for 2 hours. The xylenol orange test is repeated, and more amide added if necessary. Once the absence of free dysprosium is established by the xylenol orange test, the mixture is cooled to 50-60°C, and the pH is checked. If necessary, warm (40-50°C) 1 N NaOH may be used to adjust the pH of the mixture to 6.0-6.5.

The solution, colorless to bright yellow, is filtered into an Erlenmeyer flask. To the stirred solution, at room temperature, is added an equal volume (ca. 75 mL) of acetone. Colorless crystals are deposited over 4-24 hours. The crystals are collected, washed with 15 mL acetone, and dried in vacuo (1 mmHg, 60-80°C) for 12-24 hours. After cooling to room temperature, 25.3 g (80%) Dy DTPA-bismethylamide trihydrate (Dy DTPA-BMA) is obtained.

EXAMPLE 15

Formulation of Dy DTPA-bismethylamide and NaCa DTPA-bismethylamide

To a 50 mL volumetric flask was added solid Dy DTPA-BMA (25.3 g, 40 mmol) and 25 mL water. The mixture was heated to 55-60°C with stirring until all solids had dissolved. To the warm solution was added NaCa DTPA-bismethylamide (1.0 g, 2 mmol) with stirring. After all of the solids had dissolved, the pH 6.0 lemon-colored solution was cooled to room temperature, diluted to 50 mL with water, and sterile filtered into serum vials to yield a 800 mM solution.

EXAMPLE 16

DTPA-bisethylamide Dihydrate

DTPA-bisanhydride (50.0 g 140 mmol) was added in portions over 30 minutes to an ice-cold stirred solution of 70% ethylamine (63.1 mL, 780 mmol). Water (30 mL) was added and the mixture was stirred an additional 12 hours at ambient temperature. The mixture was concentrated under reduced pressure to an oil, diluted with water (100 mL), and adjusted to pH 2.5 with concentrated Hcl. The tan crystals which formed were collected and recrystallized from ethanol to give colorless crystals of DTPA-bisethylamide dihydrate. Yield 44.2 g (71%), mp 105-109°C. $^1H$ NMR (250 MHz, $D_2O$) $\delta$ 3.68 (s, 4 H), 3.56 (s, 4 H), 3.49 (s, 2 H), 3.19 (m, 4 H), 3.06 (m, 8 H), 0.91 (t, J = 7.0 Hz, 6 H).

| | | | |
|---|---|---|---|
| Anal. Calcd. for $C_{18}H_{37}N_5O_{10}$: | C, 44.71 | H, 7.71 | N, 14.48 |
| Found: | 44.64 | 7.85 | 14.19 |

EXAMPLE 17

Gd DTPA-bisethylamide Trihydrate

The Gd DTPA-bisethylamide trihydrate was prepared in situ in quantitative yield. A mixture of DTPA-bisethylamide dihydrate (12.1 g, 25.0 mmol) and $Gd_2O_3$ (4.53 g, 12.5 mmol) in water (30 mL) was refluxed for

5 hours. The solution was adjusted to pH 6.5 with 1 M NaOH. colorless crystals of Gd DTPA-bisethylamide trihydrate soon formed, mp > 200°C. Positive ion FAB MS: 603 $(M+H)^+$.

| | | | |
|---|---|---|---|
| Anal. Calcd. for $C_{18}H_{36}GdN_5O_{11}$: | C, 32.97 | H, 5.53 | N, 10.68 |
| Found: | 33.39 | 5.58 | 10.55 |

Replacing $Gd_2O_3$ in the above procedure by $Yb_2O_3$ or $La_2O_3$, the corresponding Yb or La chelates of DTPA-bisethylamide are produced.

EXAMPLE 18

DTPA-bispropylamide Dihydrate

The title compound is synthesised analogously to the bismethylamide and bisethylamide dihydrates of Examples 1 and 16.

In addition, since propylamine and the higher alkylamines are neat liquids at room temperature, it is possible to prepare these DTPA-bisalkylamides by direct reaction of the DTPA anhydride and the amine in an appropriate neat organic solvent and the removal of aqueous media should, in theory, increase the yield of diamide through elimination of hydrolysis of the dianhydride.

EXAMPLE 19

Gd DTPA-bispropylamide

The title compound is prepared analogously to the Gd DTPA-bisamide chelates of Examples 2 and 17.

EXAMPLE 20

Acute Toxicity

Acute toxicity ($LD_{50}$) values were determined for compositions comprising 500 Mm Gd DTPA-BMA alone or together with 25 mM of a zinc, magnesium, iron or calcium chelate of DTPA-BMA, for compositions comprising 500 mM Gd DTPA-APD alone or together with 25 mM of the calcium chelate of DTPA-APD, and, for further comparison, for compositions comprising 500 mM Gd DTPA-dimeglumine alone or together with 25 mM of a calcium chelate of DTPA or BTPA-BMA. The acute toxicities were determined by iv administration of the compositions to Swiss Webster mice.

| Gd Chelate | Added chelate | $LD_{50}$ (iv mice, mmol/kg) |
|---|---|---|
| Gd DTPA * | None | 5.5 |
| Gd DTPA * | Ca DTPA ++ | 4.5 |
| Gd DTPA * | Ca DTPA-BMA+ | 4.4 |
| Gd DTPA-BMA | None | 15.0 |
| Gd DTPA-BMA | Ca DTPA-BMA + | 34.4 |
| Gd DTPA-BMA | Mg DTPA-BMA + | 2.6 |
| Gd DTPA-BMA | Zn DTPA-BMA + | 15.8 |
| Gd DTPA-BMA | Fe DTPA-BMA | 12.8 |
| Gd DTPA-APD | None | 13.8 |
| Gd DTPA-APD | Ca DTPA-APD + | 44.0 |

* as the dimeglumine salt

\+ as the sodium salt

++ as the trisodium salt

The results set forth above clearly show the unpredicted and surprising reduction in contrast medium toxicity achievable using the present invention.

By comparison with the above $LD_{50}$ values, it may be noted that the $LD_{50}$ values for the iodinated X-ray contrast agents meglumine diatrizoate and iopamidol are respectively 18 and 25.4 (mice, rats).

EXAMPLE 21

Renal Fluoroscopy Study

After localization of the kidneys by portable fluoroscopy with sodium diatrizoate in a mongrel dog, the iodinated agent was allowed to clear. After renal clearance was complete, a dose of 1.5 mmol/kg of Gd DTPA-BMA yielded CT enhancement.

EXAMPLE 22

X-ray Attenuation Properties

The following data was obtained on a GE9800 clinical CT machine. Serial dilutions of iohexol, Gd DTPA-BMA, and Dy DTPA-BMA were placed in test tubes and imaged at the indicated energies. Region of interest Intensity values were determined. The results are shown in Tables A, B and C and in the corresponding, respective, plots of Figures 1, 2 and 3 of the accompanying drawings.

**TABLE A**

Iohexol, Gd DTPA-BMA and Dy DTPA-BMA

CT Scans, 80 keV

| Conc. mM | Gd DTPA-BMA | Dy DTPA-BMA | Iohexol/I* |
|---|---|---|---|
| 25 | | | 168 |
| 50 | 459 | 424 | 335 |
| 100 | 802 | 770 | 589 |
| 200 | 1485 | 1379 | 1023 |
| 300 | 2136 | 2003 | 1023 |
| 350 | | | 1023 |
| 400 | 2773 | 2591 | |
| 500 | 3071 | 3071 | |

* Concentration, mM of iodine

**TABLE B**

Iohexol, Gd DTPA-BMA and Dy DTPA-BMA

CT Scans, 120 keV

| Conc. mM | Gd DTPA-BMA | Dy DTPA-BMA | Iohexol/I* |
|---|---|---|---|
| 25 | | | 101 |
| 50 | 342 | 324 | 223 |
| 100 | 637 | 653 | 402 |
| 200 | 1234 | 1221 | 744 |
| 300 | 1799 | 1791 | 1016 |
| 350 | | | 1024 |
| 400 | 2340 | 2334 | |
| 500 | 2793 | 2781 | |

* Concentration, mM of iodine

**TABLE C**

Iohexol, Gd DTPA-BMA and Dy DTPA-BMA

CT Scans, 140 keV

| Conc. mM | Gd DTPA-BMA | Dy DTPA-BMA | Iohexol/I* |
|---|---|---|---|
| 25 | | | 87 |
| 50 | 306 | 293 | 199 |
| 100 | 586 | 606 | 362 |
| 200 | 1156 | 1144 | 675 |
| 300 | 1672 | 1681 | 926 |
| 350 | | | 1008 |
| 400 | 2173 | 2185 | |
| 500 | 2572 | 2605 | |

* Concentration, mM of iodine

EXAMPLE 23

Viscosities

The viscosity of Gd DTPA-bismethylamide was determined and is shown together with viscosities for conventional iodinated X-ray contrast agents in Table D below:

**TABLE D**

| Compound | | Conc., mM | Viscosity, cps (mPa.s) 20°C | 37°C |
|---|---|---|---|---|
| Gd DTPA-BMA | | 640 | 2.0 | 1.4 |
| Iohexol, | 180 mgI/ml | 473 | 3.1 | 2.0 |
| | 240 mgI/ml | 631 | 5.8 | 3.4 |
| | 300 mgI/ml | 788 | 11.8 | 6.3 |
| | 350 mgI/ml | 920 | 20.4 | 10.4 |
| Diatrizoate, | Sodium | 394 (25%) | 1.6 | 1.2 |
| | | 786 (50%) | 3.3 | 2.3 |
| Diatrizoate, | Meglumine | 371 (30%) | 1.9 | 1.4 |
| | | 742 (60%) | 6.2 | 4.1 |

## Claims

1. A diagnostic imaging contrast medium comprising a chelate of a 6-carboxymethyl-3,9-bis(carbamoylmethyl)3,6,9-triazaundecanedioic acid with a cation selected from paramagnetic metal ions, lanthanide ions and heavy metal ions and a toxicity reducing amount of a calcium chelate of a 6-carboxymethyl-3,9-bis(carbamoylmethyl)-3,6,9-triazaundecanedioic acid, said acids not carrying any alkoxyalkyl substituents.

2. A medium as claimed in claim 1 comprising a chelate of a said selected cation with a compound of formula I

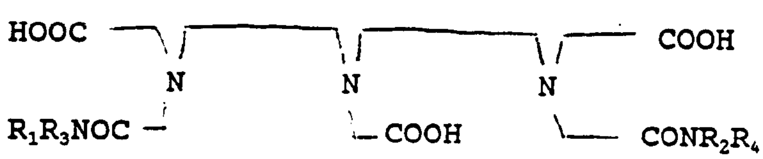

(I)

(wherein $R_1$, $R_2$, $R_3$ and $R_4$, are each independently hydrogen, $C_{1-6}$ alkyl, hydroxy $C_{1-6}$ alkyl or polyhydroxy

$C_{1-18}$ alkyl) together with a calcium chelate of a compound of formula I as hereinbefore defined.

3. A medium as claimed in claim 2 wherein said chelates are chelates of compounds of formula I as defined in claim 2 wherein $R_3$ and $R_4$ are hydrogen.

4. A medium as claimed in claim 3 wherein said chelates are chelates of compounds of formula I as defined in claim 2 wherein $R_3$ and $R_4$ are hydrogen and $R_1$ and $R_2$ are each hydrogen or $C_{1-6}$ alkyl.

5. A medium as claimed in claim 2 wherein said chelates are chelates of compounds of formula I as defined in claim 2 wherein $R_1$ and $R_2$ are each $C_{1-6}$ mono- or polyhydroxy alkyl and $R_3$ and $R_4$ are each hydrogen or $C_{1-6}$ alkyl.

6. A medium as claimed in claim 2 wherein said chelates are chelates of compounds of formula I as defined in claim 2 wherein $R_1$ and $R_2$ are each hydrogen or dihydroxypropyl and $R_3$ and $R_4$ are each hydrogen or methyl.

7. A medium as claimed in claim 2 wherein said chelates are chelates of 6-carboxymethyl-3,9-bis (methylcarbamoylmethyl)-3,6,9-triazaundecanedioic acid; 6-carboxymethyl-3,9-bis(N-methyl-N-2,3-dihydroxypropyl-carbamoylmethyl)-3,6,9-triazaundecanedioic acid; or 6-carboxymethyl-3,9-bis(2,3-dihydroxypropyl-carbamoyl-methyl)-3,6,9-triazaundecanedioic acid.

8. A medium as claimed in any one of claims 1 to 7 wherein said calcium and said selected ion chelates are chelates of the same chelating moiety.

9. A medium as claimed in claim 2 comprising as said chelates of calcium and said selected cation chelates of 6-carboxymethyl-3,9-bis(methylcarbamoylmethyl)-3,6,9-triazaundecanedioic acid.

10. A medium as claimed in claim 9 wherein said selected cation is a gadolinium cation.

11. A medium as claimed in claim 9 wherein said selected cation is a dysprosium cation.

12. A magnetic resonance imaging contrast medium as claimed in any one of claims 1 to 11 wherein said selected ion is a paramagnetic metal ion.

13. A medium as claimed in claim 12 wherein said paramagnetic metal ion is $Gd^{3+}$ or $Dy^{3+}$.

14. An X-ray imaging contrast medium as claimed in any one of claims 1 to 11 wherein said selected ion is a lanthanide ion or an ion of another metal having an atomic number of at least 50.

15. A medium as claimed in claim 14 wherein said selected ion is an ion of La, Yb, Dy or Gd.

16. A medium as claimed in any one of claims 1 to 15 wherein the molar ratio of said calcium and selected ion chelates is in the range 1:200 to 1:5.

17. A medium as claimed in claim 16 wherein the molar ratio of said calcium and selected ion chelates is in the range 1:200 to 1:10.

18. A medium as claimed in claim 16 wherein the molar ratio of said calcium and selected ion chelates is in the range 1:100 to 1:20.

19. A medium as claimed in claim 16 wherein the molar ratio of said calcium and selected ion chelates is about 1:20.

20. A medium as claimed in any one of claims 1 to 19 wherein said selected ion chelate is present at a concentration of 0.001 to 5.0 moles/litre.

21. A medium as claimed in claim 20 wherein said selected ion chelate is present at a concentration of 0.1 to 2 moles/litre.

22. A medium as claimed in claim 20 wherein said selected ion chelate is present at a concentration of 0.1 to 1.2 moles/litre.

**23.** A medium as claimed in claim 20 wherein said selected ion chelate is present at a concentration of 0.5 to 1.2 moles/litre.

**24.** A medium as claimed in any one of claims 1 to 23 comprising said chelates in solution in a sterile physiologically tolerable aqueous carrier medium.

**25.** A method of generating an image of a human or animal body, said method comprising administering to said body a contrast medium as claimed in any one of claims 1 to 24 and generating an image of at least a part of said body.

**26.** A method as claimed in claim 25 wherein said medium is administered at a dosage of 0.001 to 5 mmoles of said selected ion chelate/kg bodyweight.

**27.** A method as claimed in either one of claims 25 and 26 wherein said medium is administered at a dosage of from 0.001 to 1 mmoles of said calcium chelate/kg bodyweight.

**28.** A process for the preparation of a contrast medium as claimed in any one of claims 1 to 24 which process comprises reacting in aqueous solution a said 6-carboxymethyl-3,9-bis(carbamoylmethyl)-3,6,9-triazaundecanedioic acid, a soluble calcium compound and a soluble compound of a paramagnetic metal species, a lanthanide or a heavy metal.

**29.** A process for the preparation of a contrast medium as claimed in any one of claims 1 to 24 which process comprises admixing at least one said calcium chelate together with at least one said selected ion chelate.

**30.** The use of a 6-carboxymethyl-3,9-bis (carbamoylmethyl)-3,6,9-triazaundecanedioic acid for the manufacture of a diagnostic imaging agent as claimed in any of claims 1 to 24.

**31.** The use of a calcium chelate of a 6-carboxymethyl-3,9-bis(carbamoylmethyl)-3,6,9-triazaundecanedioic acid for the manufacture of a diagnostic imaging agent as claimed in any of claims 1 to 24.

**32.** The use of a chelate of a 6-carboxymethyl-3,9-bis (carbamoylmethyl)-3,6,9-triazaundecanedioic acid with an ion selected from paramagnetic metal ions, lanthanide ions and other heavy metal ions for the manufacture of a diagnostic imaging agent as claimed in any of claims 1 to 24.

## Patentansprüche

**1.** Kontrastmedium zur diagnostischen Bildgebung, umfassend ein Chelat einer 6-Carboxymethyl-3,9-bis(carbamoylmethyl)3,6,9-triazaundecandicarbonsäure mit einem Kation, ausgewählt unter paramagnetischen Metallionen, Lanthanid-Ionen und Schwermetallionen, und eine die Toxizität verringernde Menge eines Calciumchelats einer 6-Carboxymethyl-3,9-bis(carbamoylmethyl)-3,6,9-triazaundecandicarbonsäure, wobei die Säuren keine Alkoxyalkyl-Substituenten tragen.

**2.** Medium nach Anspruch 1, umfassend ein Chelat eines Kations mit einer Verbindung der Formel I

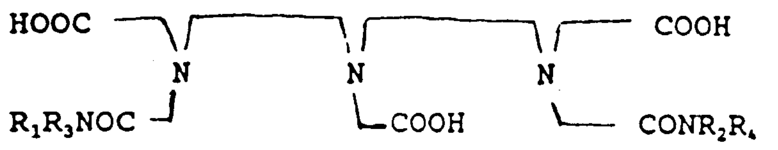

(I)

(worin $R_1$, $R_2$, $R_3$ und $R_4$ jeweils unabhängig voneinander ein Wasserstoffatom, eine $C_{1-6}$-Alkylgruppe, eine Hydroxy-$C_{1-6}$-alkyl-oder Polyhydroxy-$C_{1-18}$-alkylgruppe bedeuten) zusammen mit einem Calciumchelat einer Verbindung der oben angegebenen Formel I.

**3.** Medium nach Anspruch 2, worin die Chelate Chelate von Verbindungen der Formel I gemäß Anspruch 2 sind, wobei $R_3$ und $R_4$ für ein Wasserstoffatom stehen.

4. Medium nach Anspruch 3, worin die Chelate Chelate von Verbindungen der Formel I gemäß Anspruch 2 sind, worin $R_3$ und $R_4$ für ein Wasserstoffatom stehen und $R_1$ und $R_2$ jeweils für ein Wasserstoffatom oder eine $C_{1-6}$-Alkylgruppe stehen.

5. Medium nach Anspruch 2, worin die Chelate Chelate von Verbindungen der Formel I gemäß Anspruch 2 sind, worin $R_1$ und $R_2$ jeweils für eine $C_{1-6}$-Mono- oder Polyhydroxyalkylgruppe stehen und $R_3$ und $R_4$ jeweils für ein Wasserstoffatom oder eine $C_{1-6}$-Alkylgruppe stehen.

6. Medium nach Anspruch 2, worin die Chelate Chelate von Verbindungen der Formel I gemäß Anspruch 2 sind, worin $R_1$ und $R_2$ jeweils für ein Wasserstoffatom oder eine Dihydroxypropylgruppe stehen und $R_3$ und $R_4$ jeweils für ein Wasserstoffatom oder eine Methylgruppe stehen.

7. Medium nach Anspruch 2, worin die Chelate Chelate von 6-Carboxymethyl-3,9-bis(methylcarbamoylmethyl)-3,6,9-trizaundecandicarbonsäure; 6-Carboxymethyl-3,9-bis(N-methyl-N-2,3-dihydroxypropyl-carbamoylmethyl)-3,6,9-triazaundecandicarbonsäure; oder 6-Carboxymethyl-3,9-bis(2,3-dihydroxypropylcarbamoyl-methyl)-3,6,9-triazaundecandicarbonsäure sind.

8. Medium nach einem der Ansprüche 1 bis 7, worin die Chelate von Calcium und dem ausgewählten Ion Chelate des gleichen chelatbildenden Restes sind.

9. Medium nach Anspruch 2, umfassend als Chelate von Calcium und dem ausgewählten Kation Chelate von 6-Carboxymethyl-3,9-bis(methylcarbamoylmethyl)-3,6,9-triazaundecandicarbonsäure.

10. Medium nach Anspruch 9, worin das ausgewählte Kation ein Gadoliniumkation ist.

11. Medium nach Anspruch 9, worin das ausgewählte Kation ein Dysprosiumkation ist.

12. Kontrastmedium für die Kernspinresonanztomographie nach einem der Ansprüche 1 bis 11, worin das ausgewählte Ion ein paramagnetisches Metallion ist.

13. Medium nach Anspruch 12, worin das paramagnetische Metallion $Gd^{3+}$ oder $Dy^{3+}$ ist.

14. Röntgenkontrastmedium nach einem der Ansprüche 1 bis 11, worin das ausgewählte Ion ein Lanthanidion oder ein Ion eines anderen Metalls mit einer Ordnungszahl von wenigstens 50 ist.

15. Medium nach Anspruch 14, worin das ausgewählte Ion ein La-, Yb-, Dy- oder Gd-Ion ist.

16. Medium nach einem der Ansprüche 1 bis 15, worin das molare Verhältnis von Calciumchelat zum Chelat des ausgewählten Ions im Bereich von 1:200 bis 1:5 liegt.

17. Medium nach Anspruch 16, worin das molare Verhältnis von Calciumchelat zum Chelat des ausgewählten Ions im Bereich von 1:200 bis 1:10 liegt.

18. Medium nach Anspruch 16, worin das molare Verhältnis von Calciumchelat zum Chelat des ausgewählten Ions im Bereich von 1:100 bis 1:20 liegt.

19. Medium nach Anspruch 16, worin das molare Verhältnis von Calciumchelat zum Chelat des ausgewählten Ions etwa 1:20 beträgt.

20. Medium nach einem der Ansprüche 1 bis 19, worin das Chelat des ausgewählten Ions in einer Konzentation von 0,001 bis 5,0 Mol/Liter vorliegt.

21. Medium nach Anspruch 20, worin das Chelat des ausgewählten Ions in einer Konzentration von 0,1 bis 2 Mol/Liter vorliegt.

22. Medium nach Anspruch 20, worin das Chelat des ausgewählten Ions in einer Konzentration von 0,1 bis 1,2 Mol/Liter vorliegt.

23. Medium nach Anspruch 20, worin das Chelat des ausgewählten Ions in einer Konzentration von 0,5 bis 1,2 Mol/Liter vorliegt.

24. Medium nach einem der Ansprüche 1 bis 23, umfassend die Chelate gelöst in einem sterilen, physiologisch verträg-lichen, wäßrigen Trägermedium.

25. Verfahren zur Erzeugung einer Abbildung eines menschlichen oder tierischen Körpers, wobei das Verfahren die Verabreichung eines Kontrastmediums gemäß einem der Ansprüche 1 bis 24 an den Körper und die Erzeugung einer Abbildung von wenigstens einem Teil des Körpers umfaßt.

26. Verfahren nach Anspruch 25, worin das Medium in einer Dosis von 0,001 bis 5 mMol des Chelats des ausgewählten Ions/kg Körpergewicht verabreicht wird.

27. Verfahren nach einem der Ansprüche 25 und 26, worin das Medium in einer Dosis von 0,001 bis 1 mMol des Chelats des ausgewählten Ions/kg Körpergewicht verabreicht wird.

28. Verfahren zur Herstellung eines Kontrastmediums gemäß einem der Ansprüche 1 bis 24, wobei man in einer wäßrigen Lösung die 6-Carboxymethyl-3,9-bis(carbamoylmethyl)-3,6,9-triazaundecandicarbonsäure, eine lösliche Calciumverbindung und eine lösliche Verbindung einer paramagnetischen Metallspecies eines Lanthanids oder eines Schwermetalls umsetzt.

29. Verfahren zur Herstellung eines Kontrastmediums nach einem der Ansprüche 1 bis 24, wobei man wenigstens ein Calciumchelat mit wenigstens einem Chelat des ausgewählten Ions vermischt.

30. Verwendung einer 6-Carboxymethyl-3,9-bis(carbamoylmethyl)-3,6,9-triazaundecandicarbonsäure zur Herstellung eines Mittels zur diagnostischen Bildgebung nach einem der Ansprüche 1 bis 24.

31. Verwendung eines Chelats einer 6-Carboxymethyl-3,9-bis(carbamoylmethyl)-3,6,9-triazaundecandicarbonsäure zur Herstellung eines Mittels zur diagnostischen Bildgebung nach einem der Ansprüche 1 bis 24.

32. Verwendung eines Chelats einer 6-Carboxymethyl-3,9-bis(carbamoylmethyl)-3,6,9-triazaundecandicarbonsäure mit einem Ion, ausgewählt unter paramagnetischen Metallionen, Lanthanid-Ionen und anderen Schwermetallionen, zur Herstellung eines Mittels zur diagnostischen Bildgebung nach einem der Ansprüche 1 bis 24.

## Revendications

1. Agent de contraste pour l'imagerie de diagnostic, comprenant un chélate d'un acide 6-carboxyméthyl-3,9-bis(carbamoylméthyl)-3,6,9-triazaundécanedioïque avec un cation sélectionné parmi les ions de métaux paramagnétiques, les ions lanthanide et les ions de métaux lourds et une quantité réduisant la toxicité d'un chélate de calcium d'un acide 6-carboxyméthyl-3,9-bis(carbamoylméthyl)-3,6,9-triazaundécanedioïque, ces acides ne portant pas de substituant alcoxyalcoyle.

2. Agent suivant la revendication 1, comprenant un chélate du cation sélectionné avec un composé de formule I

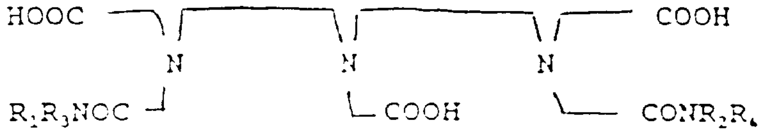

(I)

(dans laquelle $R_1$, $R_2$, $R_3$ et $R_4$ sont chacun indépendamment l'hydrogène, alcoyle ayant de 1 à 6 atomes de carbone, hydroxyalcoyle ayant de 1 à 6 atomes de carbone ou polyhydroxyalcoyle ayant de 1 à 18 atomes de carbone) avec un chélate de calcium d'un composé de formule I tel que défini ci-dessus.

3. Agent suivant la revendication 2, dans lequel les chélates sont des chélates de composés de formule I tels que définis à la revendication 2, dans laquelle $R_3$ et $R_4$ sont l'hydrogène.

4. Agent suivant la revendication 3, dans lequel les chélates sont des chélates de composés de formule I tels que définis dans la revendication 2, dans laquelle $R_3$ et $R_4$ sont l'hydrogène et $R_1$ et $R_2$ sont chacun

de l'hydrogène ou alcoyle ayant de 1 à 6 atomes de carbone.

5. Agent suivant la revendication 2, dans lequel les chélates sont des chélates de composés de formule I tels que définis dans la revendication 2, dans laquelle $R_1$ et $R_2$ sont chacun des alcoyles monohydroxylés ou polyhydroxylés ayant de 1 à 6 atomes de carbone et $R_3$ et $R_4$ sont chacun de l'hydrogène ou alcoyle ayant de 1 à 6 atomes de carbone.

6. Agent suivant la revendication 2, dans lequel les chélates sont des chélates de composés de formule I tels que définis à la revendication 2, dans laquelle $R_1$ et $R_2$ sont chacun l'hydrogène ou dihydroxypropyle et $R_3$ et $R_4$ sont chacun l'hydrogène ou méthyle.

7. Agent suivant la revendication 2, dans lequel les chélates sont des chélates d'acide 6-carboxyméthyl-3,9-bis (méthylcarbamoylméthyl)-3,6,9-triazaundécanedioïque; d'acide 6-carboxyméthyl-3,9-bis(N-méthyl-N-2,3-dihydroxypropyl-carbamoylméthyl)-3,6,9-triazaundécanedioïque; ou d'acide 6-carboxyméthyl-3,9-bis(2,3-dihydroxypropyl-carbamoylméthyl)-3,6,9-triazaundécanedioïque.

8. Agent suivant l'une quelconque des revendications 1 à 7, dans lequel les chélates de calcium et de l'ion sélectionné sont des chélates ayant le même radical de chélation.

9. Agent suivant la revendication 2, comprenant comme chélate de calcium et du cation sélectionné, des chélates de l'acide 6-carboxyméthyl-3,9-bis(méthylcarbamoylméthyl)-3,6,9-triazaundécanedioïque.

10. Agent suivant la revendication 9, dans lequel le cation sélectionné est le cation de gadolinium.

11. Agent suivant la revendication 9, dans lequel le cation sélectionné est le cation dysprosium.

12. Agent de contraste pour l'imagerie par résonance magnétique suivant l'une quelconque des revendications 1 à 11, dans lequel l'ion sélectionné est un ion de métal paramagnétique.

13. Agent suivant la revendication 12, dans lequel l'ion de métal paramagnétique est $Gd^{3+}$ ou $Dy^{3+}$.

14. Agent de contraste pour l'imagerie par rayons X suivant l'une quelconque des revendications 1 à 11, dans lequel l'ion sélectionné est un ion lanthanide ou un ion d'un autre métal ayant un numéro atomique d'au moins 50.

15. Agent suivant la revendication 14, dans lequel l'ion sélectionné est un ion La, Yb, Dy ou Gd.

16. Agent suivant l'une quelconque des revendications 1 à 15, dans lequel le rapport molaire du chélate de calcium au chélate de l'ion sélectionné est compris entre 1:200 et 1:5.

17. Agent suivant la revendication 16, dans lequel le rapport molaire du chélate de calcium au chélate de l'ion sélectionné est compris entre 1:200 et 1:10.

18. Agent suivant la revendication 16, dans lequel le rapport molaire du chélate de calcium ou du chélate de l'ion sélectionné est compris entre 1:100 et 1:20.

19. Agent suivant la revendication 16, dans lequel le rapport molaire du chélate de calcium au chélate de l'ion sélectionné est de 1:20 environ.

20. Agent suivant l'une quelconque des revendications 1 à 19, dans lequel le chélate de l'ion sélectionné est présent en une concentration de 0,001 à 5,0 moles/litre.

21. Agent suivant la revendication 20, dans lequel le chélate de l'ion sélectionné est présent en une concentration de 0,1 à 2 moles/litre.

22. Agent suivant la revendication 20, dans lequel le chélate de l'ion sélectionné est présent en une concentration de 0,1 à 1,2 moles/litre.

23. Agent suivant la revendication 20, dans lequel le chélate de l'ion sélectionné est présent en une concentration de 0,5 à 1,2 moles/litre.

**24.** Agent suivant l'une des revendications 1 à 23, comprenant les chélates en solution dans un support aqueux acceptable physiologiquement.

**25.** Procédé de formation de l'image d'un organisme humain ou animal, qui consiste à administrer à cet organisme un agent de contraste suivant l'une quelconque des revendications 1 à 24 et à former une image d'au moins une partie de cet organisme.

**26.** Procédé suivant la revendication 25, qui consiste à administrer le milieu à une posologie de 0,001 à 5 mmoles du chélate d'ion sélectionné/kg de poids corporel.

**27.** Procédé suivant l'une des revendications 25 ou 26, qui consiste à administrer l'agent à une posologie de 0,001 à 1 mmole du chélate de calcium/kg de poids corporel.

**28.** Procédé de préparation d'un milieu de contraste suivant l'une quelconque des revendications 1 à 24, qui consiste à mettre à réagir en solution aqueuse un acide 6-carboxyméthyl-3,9-bis(carbamoylméthyl)-3,6,9-triazaundécanedioïque, un composé soluble de calcium et un composé soluble d'une espèce métallique paramagnétique, d'un lanthanide ou d'un métal lourd.

**29.** Procédé de préparation d'un agent de contraste suivant l'une quelconque des revendications 1 à 24, qui consiste à mélanger au moins le chélate de calcium à au moins le chélate de l'ion sélectionné.

**30.** L'utilisation de l'acide 6-carboxyméthyl-3,9-bis (carbamoylméthyl)-3,6,9-triazaundécanedioïque pour la fabrication d'un agent pour l'imagerie de diagnostic tel que revendiqué à l'une des revendications 1 à 24.

**31.** L'utilisation d'un chélate de calcium de l'acide 6-carboxyméthyl-3,9-bis(carbamoylméthyl)-3,6,9-triazaundécanedioïque pour la fabrication d'un agent pour l'imagerie de diagnostic tel que revendiqué suivant l'une quelconque des revendications 1 à 24.

**32.** L'utilisation d'un chélate de calcium de l'acide 6-carboxyméthyl-3,9-bis(carbamoylméthyl)-3,6,9-triazaundécanedioïque avec un ion sélectionné parmi les ions de métaux paramagnétiques, les ions lanthanides et les autres ions de métaux lourds pour la fabrication d'un agent pour l'imagerie de diagnostic tel que revendiqué suivant l'une quelconque des revendications 1 à 24.

FIG.1

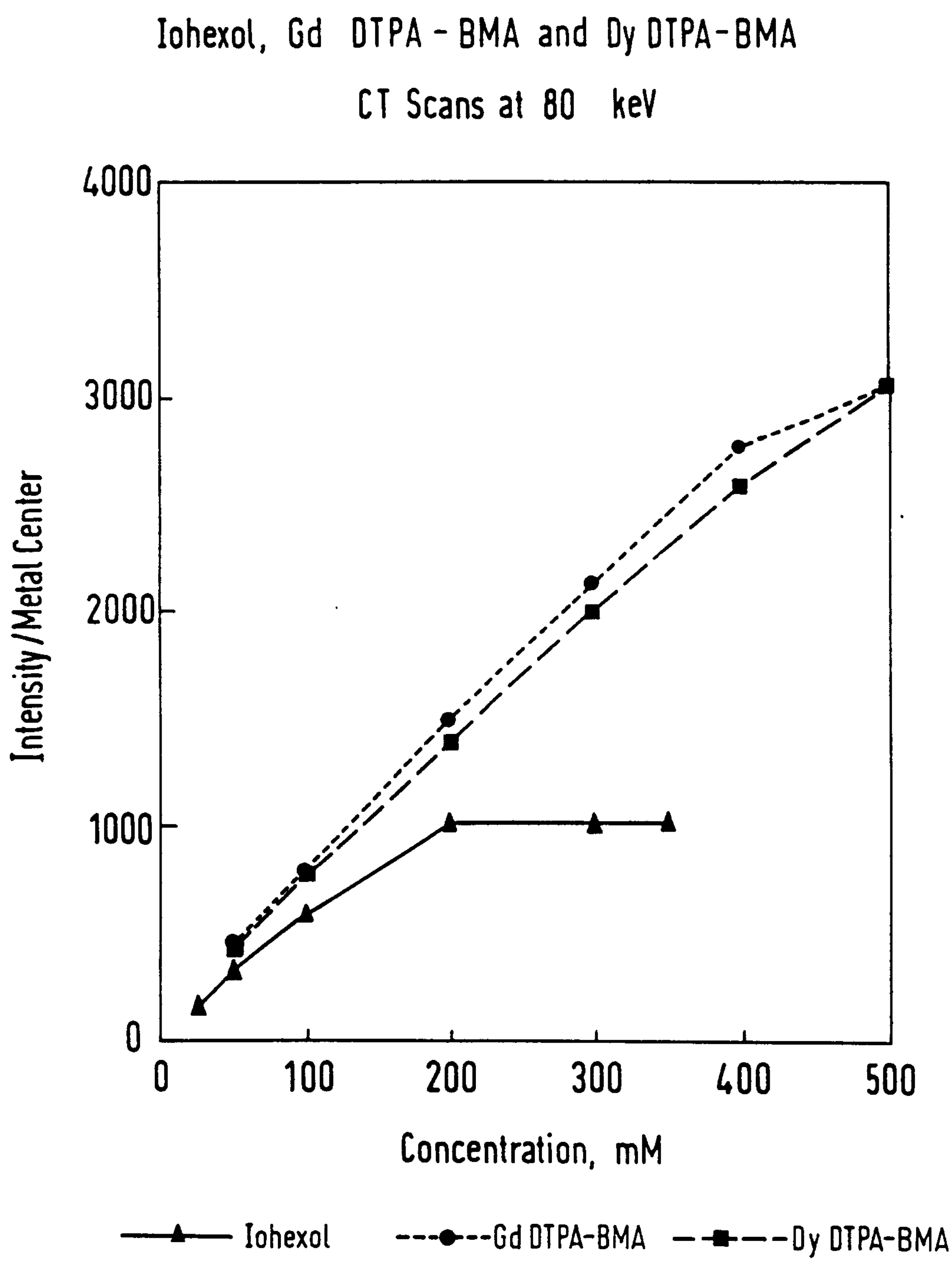
Iohexol, Gd DTPA - BMA and Dy DTPA-BMA
CT Scans at 80 keV
4000
3000
2000
1000
0
0
100
200
300
400
500
Intensity/Metal Center
Concentration, mM
Iohexol
Gd DTPA-BMA
Dy DTPA-BMA

FIG. 2

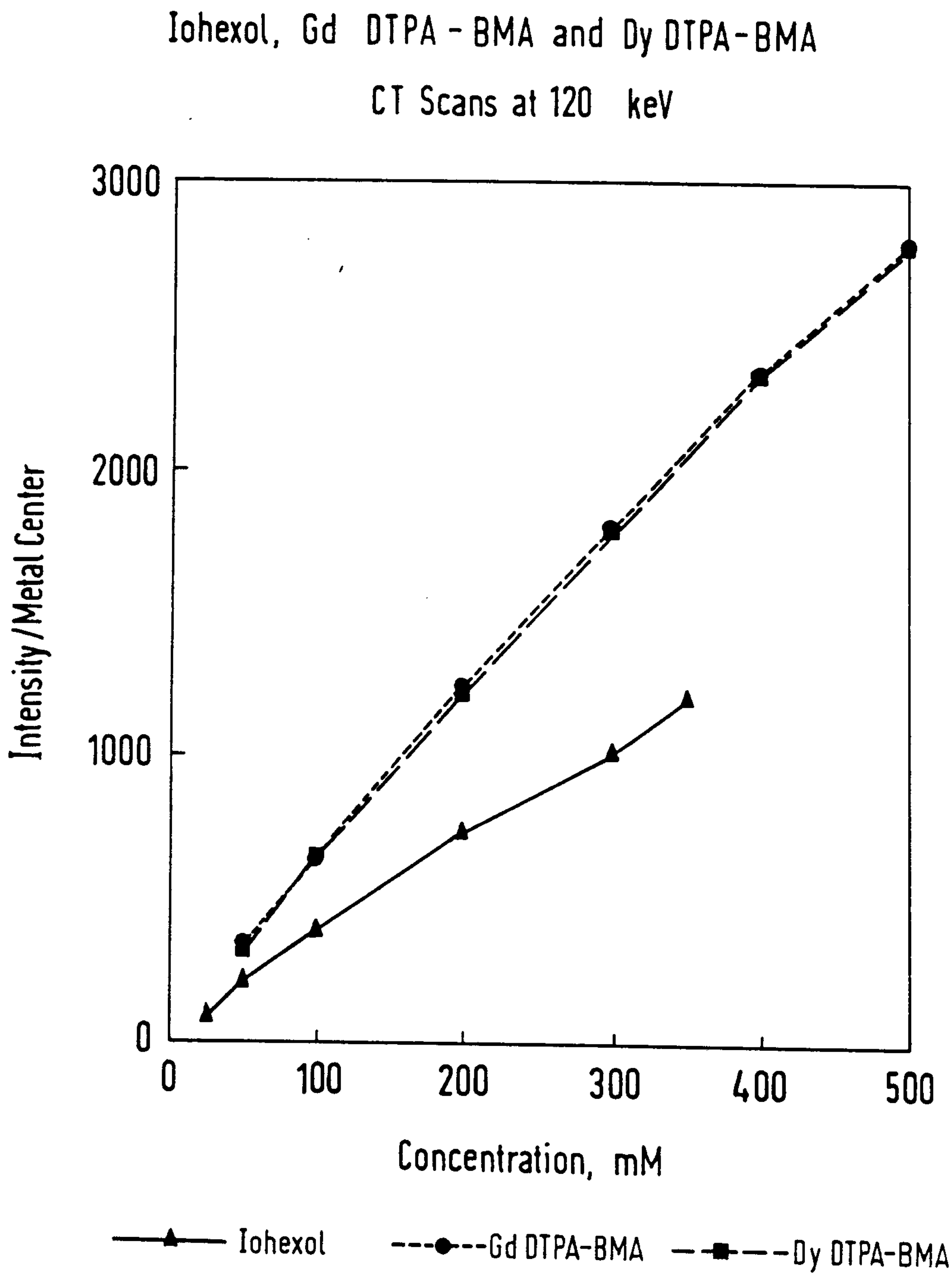
Iohexol, Gd DTPA - BMA and Dy DTPA-BMA
CT Scans at 120 keV
3000
2000
1000
0
Intensity/Metal Center
0
100
200
300
400
500
Concentration, mM
Iohexol
Gd DTPA-BMA
Dy DTPA-BMA

FIG.3

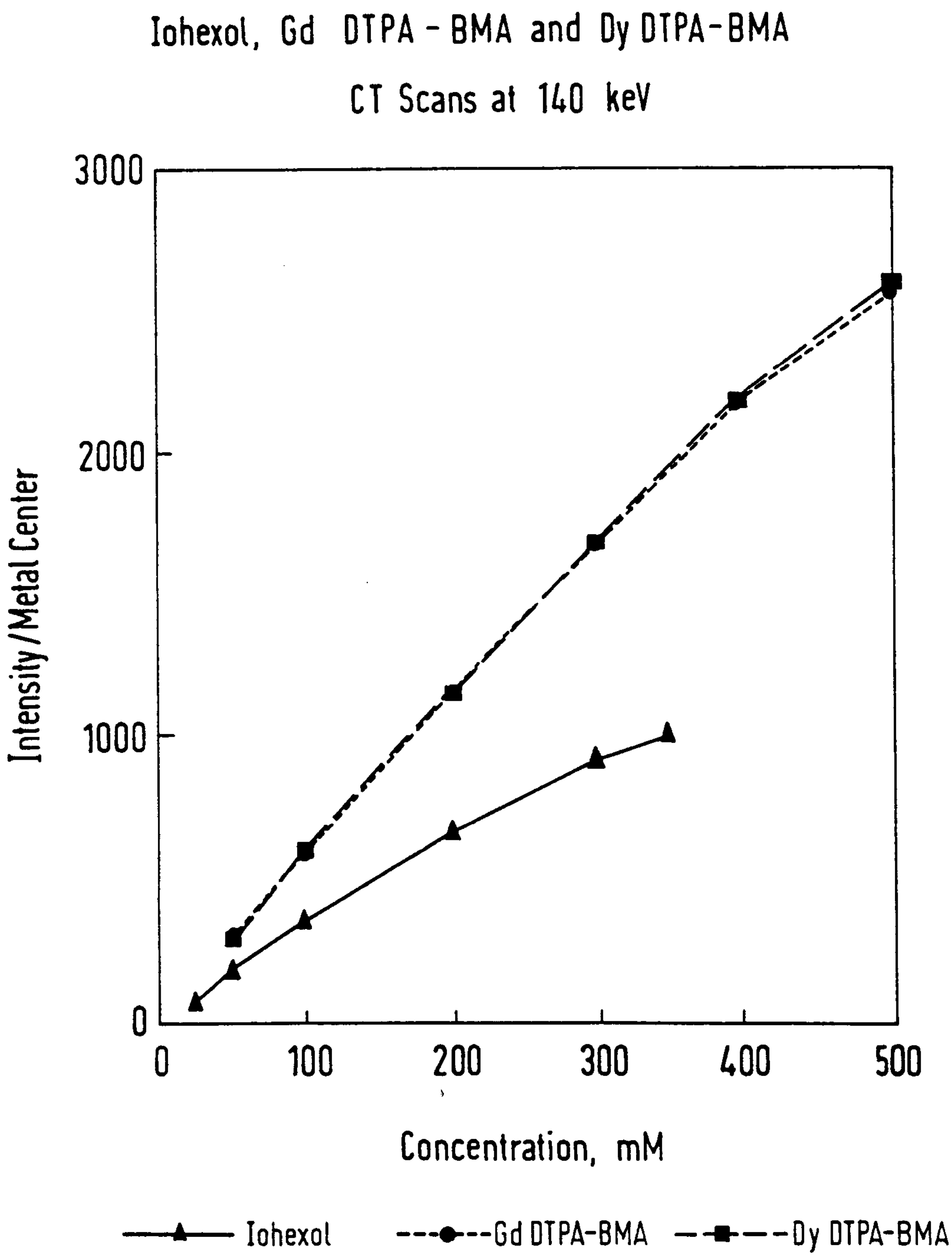
Iohexol, Gd DTPA - BMA and Dy DTPA-BMA
CT Scans at 140 keV
3000
2000
1000
0
Intensity/Metal Center
0
100
200
300
400
500
Concentration, mM
Iohexol
Gd DTPA-BMA
Dy DTPA-BMA